# EUROPEAN PATENT SPECIFICATION

(11) **EP 2 091 367 B1**
(45) Date of publication and mention of the grant of the patent: **28.08.2013**
(21) Application number: 07835241.6
(22) Date of filing: 28.11.2007
(51) Int. Cl.: A42B 1/06, A61F 11/14

(54) **HEADPIECE FOR USE IN ASSOCIATION WITH A HEARING CAP**
KOPFTEIL FÜR DIE VERWENDUNG IN VERBINDUNG MIT EINER HÖRMÜTZE
CASQUE À UTILISER AVEC UN PROTÈGE-OREILLES

(30) Priority: 11.12.2006 SE 0602668
(43) Date of publication of application: 26.08.2009
(73) Proprietor: Sperian Hearing Protection, LLC, San Diego, CA 92154 (US)
(72) Inventor: HANSSON, Fredrik, 254 58 Helsingborg (SE)
(74) Representative: Åkesson, Sten Jan-Åke
(86) International application number: PCT/SE2007/001051
(87) International publication number: WO 2008/073016

(56) References cited:
- DE-A1- 2 847 724
- US-A- 1 879 353
- US-A- 2 902 692
- US-A- 3 588 914
- US-A- 3 731 320
- US-A1- 2006 288 468

## Description

### Technical field of the invention

The present invention relates to a headpiece for use in association with a hearing cap, such as a hearing cap for a hearing protection.

### Background of the invention

A common type of hearing protection comprises two hearing caps or ear muffs which are connected to each other by a headband. The headband is adapted to be placed over the head of the user and the two caps are adapted to be arranged one on each side of the user's head so as to enclose respective external ears of the user. Each cap in turn comprises a cap shell, which usually is made of plastic, a sealing ring, which is adapted to, by being resilient, fit the head of the user, and some kind of sound-absorbing material arranged inside the cap shell. The caps are also commonly provided with a so-called bottom plate. The bottom plate is positioned between the sealing ring and the cap shell and connects the sealing ring to the cap shell. The bottom plate also commonly acts to keep the sound-absorbing material in place inside the cap shell.

The function of the hearing protection is to attenuate noise from noise sources in the surroundings so that the noise does not reach the user's ears without first being attenuated to acceptable levels.

In order to secure that adequate sound attenuation is provided the different kinds of hearing protection are classified according to different standards. The performance of the hearing protection is tested in accordance with specified test procedures and are marked with performance classifications. This way a user may purchase the hearing protection and rely on the performance classification when it comes to attenuation properties and thereby secure not to damage his/her hearing.

There are several working places where it is necessary and compulsory to use hearing protection at the same time as the work takes place outdoors. During the cold seasons, it is often necessary to wear a cap or another type of headpiece for staying warm and for protecting the user's head from wind, snow or rain. Such workplaces can be found, for example, at airports during loading/unloading, refuelling, de-icing etc, at road works, at construction sites, in the mining industry, during forestry work, gardening, harbour work, at cargo-ships and at oil rigs etc. In such places, there is a need for wearing both a protecting headpiece and a hearing protection with hearing caps. During the warmer seasons, a protecting headpiece could be desirable to protect the user from the sun and wind.

If a user wears a headpiece and then hearing caps on the top of the headpiece, the sound attenuation of the hearing cap is reduced to a far too large extent. The resulting sound attenuation will be in the order of the sound attenuation of the headpiece as such, i.e. significantly inferior and often dangerously inferior to the sound attenuation of the hearing protection. Thus, by wearing the hearing protection outside or on top of the headpiece, the user risks injuring his/her hearing. If the user instead wears a headpiece over the hearing cap, the user feels cold since air can pass under the headpiece.

In this connection DE 28 47 724 may be mentioned. This document discloses a hearing cap integrated in a headpiece in the form of a cap. This design does not permit the user to use the same hearing protection both in association with a headpiece and without. Thus, the user must have different hearing protections for different weather conditions and different circumstances. Moreover, if the user would like to work under circumstances requiring a protective headpiece but has no use for a hearing protection he also needs to bring with him a separate headpiece for this occasion.

Wearing the cap with the integrated hearing protection in accordance with DE 28 47 724 in combination with an ordinary headpiece will result in the same problems as with an ordinary hearing protection in combination with an ordinary headpiece.

US 3,731,320 discloses a liner type cap for use under a helmet. Hearing caps are held in position by retention members attached to ear depending portions of the liner, in which apertures are located.

### Summary of the invention

An object of the present invention is to provide an improvement over the prior art in aiming at solving the above problems.

The above object has been achieved by a headpiece for use in association with at least one hearing cap. The headpiece comprises a first portion, adapted to, when a user wears a hearing cap, be positioned between a sealing ring of the hearing cap and a portion of the user's head circumscribing an ear. The headpiece further comprises a second portion essentially circumscribed by the first portion. The first portion comprises a relatively air tight material forming a sealing ring in the headpiece. The second portion is formed of one or more relatively air permeable materials allowing air to move between the ear and an inner volume of the hearing cap when the user wears the hearing cap.

By providing the headpiece with a first relatively air tight portion circumscribing the user's ear and with a relatively air permeable portion essentially covering the ear, it has become possible to wear a headpiece, such as a cap or hood, with a hearing cap, such as a hearing protection or the like, and still have a satisfactory sound attenuation. The relatively air tight portion cooperates with the sealing ring of the cap of the hearing protection thereby preventing air and thereby also preventing sound waves from entering into the hearing protection. The relatively air permeable material on the other hand allows air to move between the inside of the ear and the inside of the hearing cap, thereby preventing the headpiece from acting as a membrane, which otherwise would transfer any vibration of the cap shell to the user's ear and thereby dramatically change the acoustic properties of the hearing cap.

By not allowing air to enter between the user's head and the hearing cap and by making sure that a membrane is not formed, the headpiece may be designed such that it when it is worn in association with a hearing cap does not change the acoustic properties of the hearing cap in an dissatisfactory manner. Thus, the hearing cap can maintain its hearing protection classification when it is used in combination with the headpiece in accordance with the invention.

Thus, the inventive headpiece allows the user to use the same hearing cap as he/she normally does. The hearing cap does not have to be adapted to be used in association with the headpiece. The user can remove the head piece and simply put the hearing cap back on. The user may also remove the hearing cap and still benefit from wearing the headpiece.

When the user removes the hearing cap of the hearing protection or the like, his/her ear is still protected against the cold, rain, wind, or sun. Even though the material of the second portion is air permeable in order to avoid the formation of a membrane, it has been found that it still offers a protection not only for sun, rain and heat/cold but also against wind. This might be explained by the fact that the headpiece still offers a slight air flow resistance, thereby aiding in forming a cushion of air being slightly closer to body temperature.

When the headpiece is worn without the hearing cap, the second portion permits the user to hear normally and still protects the ear from the cold, rain, wind or sun.

It may be noted that the level of air tightness of the first portion and the level of air permeability are dependent upon the specific use. For most cases the criterion is that the sound attenuation with the headpiece under the hearing cap should be the same as the sound attenuation of the hearing cap as such. If the first portion is not tight enough air, and thereby also sound, may leak into the users ear, and if the second portion is not air permeable enough it may act as a membrane thereby transmitting vibrations into the users ear. It may in this context be noted that for a textile or the like it is difficult to measure the resistance to air flow for a flow in the plane of the material. The flow path of the air, when leaking via the first portion, extends into the material of the first portion in the direction of the normal of the surface of the first portion, the flow path then continues inside the material of the first portion along the plane of the first portion, and finally leaves the first portion in the direction of the normal, wherein the distance or length of the flow path is corresponding to the width of the abutment of the sealing ring of the hearing cap onto the first portion.

The basic concept is put into use as long as the first portion is relatively air tight and the second portion is relatively air permeable.

Considering the above, the most expedient way to determine the desired level of air tightness and air permeability is to make sound attenuation tests as is well-known in the art. One such test is described briefly in the detailed description in connection with the analysis of the sound attenuation graphs.

The first portion and the second portion may form an ear portion, and wherein said headpiece may comprise two ear portions, one for each ear of the user. Such a design is especially useful for use in connection with hearing caps for hearing protection.

The first portion may form a closed loop. In this context it may be noted that the shape is not important as such as long as the first portion provides a sufficient abutment surface for the hearing cap it is to be used with.

The first portion may be adapted to, when a user wears the hearing cap, be in contact with the sealing ring of the hearing cap. This design makes it easier to secure that the desired air tightness is achieved.

The first portion may be formed of a rigid material. This is a suitable way of securing that the first portion does not affect the sound attenuation in a negative manner. It may be noted that although the first portion is said to be formed of a rigid material it is still thin such that it will be able to bend and follow the shape of the user's head. The rigidity relates especially to the behaviour of the material in the direction of the normal to the surface formed by the first portion.

The first portion may be thin such that the first portion is rigid. By forming the first portion thin it may be comparably rigid in the direction of the thickness of the material even though it is formed of a relatively elastic material.

The first portion may be air tight such that the acoustic properties of the hearing cap is substantially maintained compared to the hearing cap when worn without the headpiece. This way it is secured that the hearing protection may be used with the same safety level independently of being used as such or with the headpiece.

The second portion may be formed of one or more flexible, preferably elastic, materials adapted to, when a user wears the headpiece, accommodate the user's ear. This allows the first portion to remain flat, without any creases or the like, which would otherwise seriously affect the acoustic properties of the hearing cap. Moreover, the second portion follows the form of the user's ear and accommodates different sizes and shapes of different users' ears.

The second portion may be air permeable such that the acoustic properties of the hearing cap is substantially maintained compared to the hearing cap when worn without the headpiece. This way it is secured that the hearing protection may be used with the same safety level independently of being used as such or with the headpiece.

As discussed above one advantage of this concept is that a headpiece and a hearing protection may suitably be considered as a kit. The user may purchase the kit and knows that the same sound attenuation is achieved irrespective of if the hearing protection is used directly on his/her head or if the hearing protection is used with the specifically adapted headpiece.

### Brief description of the drawings

The present invention will be described in further details hereinafter with reference to the accompanying drawings, which shows an embodiment of the invention in form of a non-limiting example.
Fig 1 shows a user wearing a headpiece in accordance with a first embodiment of the invention.
Fig 2 shows a user wearing the headpiece of fig 1 and also wearing on top of the headpiece a hearing protection with two hearing caps.
Fig 3 shows a user wearing a headpiece in accordance with a second embodiment of the invention.
Fig 4 is a set of graphs showing the sound attenuation for cases where the user wears different kinds of common headpieces under a hearing protection in comparison with wearing the hearing protection without any headpiece and with wearing the hearing protection on top of the inventive headpiece. The sound attenuation is indicated in decibel (dB) for different frequencies.
Fig 5 shows the corresponding graphs as fig 4 but for another hearing cap.
Fig 6 shows two graphs using different elasticity of a material in the first relatively air tight portion of the headpiece.
Fig 7 shows several graphs using different thicknesses of a material in the first relatively air tight portion of the headpiece.

### Detailed description

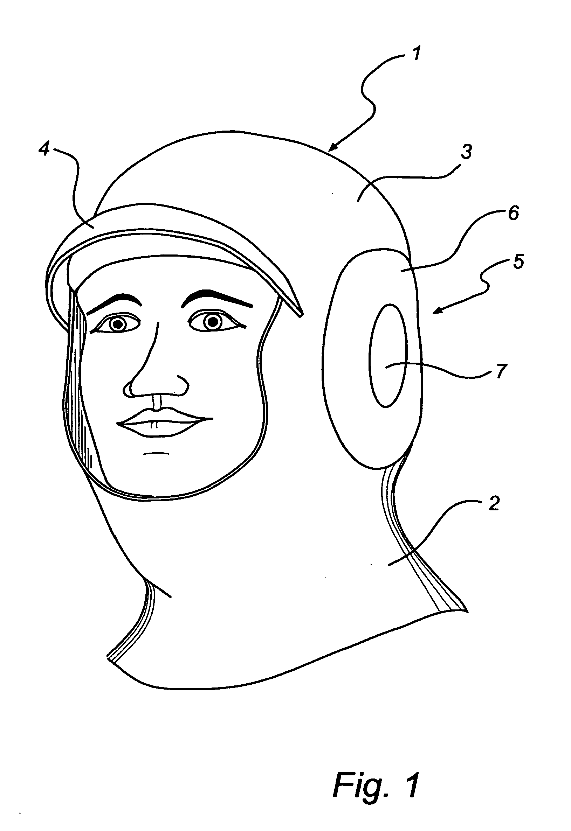

The headpiece 1 of fig 1 is formed as a hood that is pulled over the user's head. The hood 1 comprises a neck portion 2, a head portion 3, and a cap peak or visor 4. Such a headpiece 1 may be used as a ground crew cap being worn by the ground crew at an airport.

The headpiece 1 further comprises an ear portion 5. The headpiece comprises an annular first portion 6 and a second portion 7 encircled or circumscribed by the first portion 6. The second portion 7 is circumscribed by the first portion 6 on a common surface or a common plane of the hood. The first portion 6 forms a closed loop forming a sealing ring. The first portion 6 and the second portion 7 form said ear portion 5.

The headpiece 1 of fig 1 is provided with a first portion 6 and second portion 7 on both sides of the headpiece 1, i.e. the headpiece 1 is provided with two ear portions 5, one for each ear of the user.

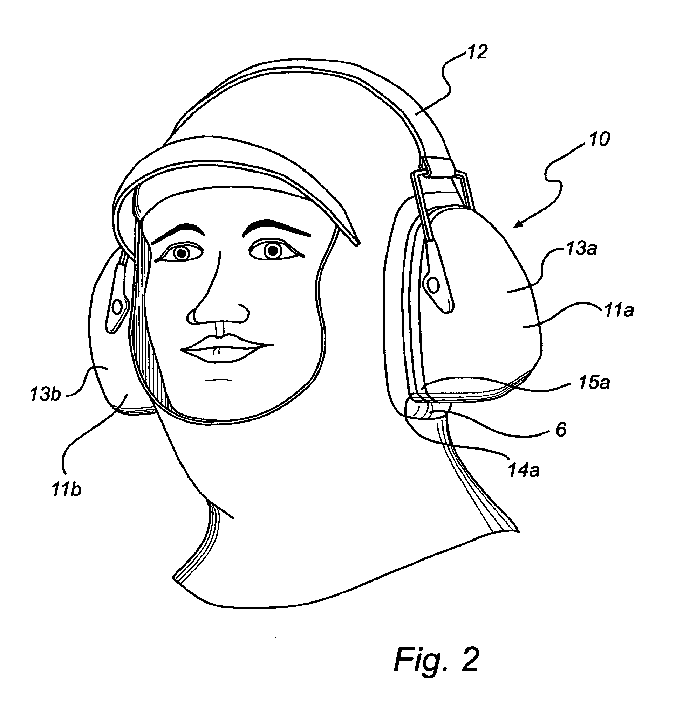

The headpiece 1 is adapted to be used in association with a single hearing cap or with a set of two hearing caps, such as in a hearing protection as shown in fig 2.

A common type of hearing protection 10 comprises two hearing caps 11 a, 11 b or ear muffs which are connected to each other by a headband 12. The headband 12 is adapted to be placed over the head of the user and the two caps 11 a, 11 b are adapted to be arranged one on each side of the user's head so as to enclose respective external ears of the user. Each cap 11 a, 11b in turn comprises a cap shell 13a, 13b, which usually is made of plastic, a sealing ring 14a (only visible at the user's left ear) which is adapted to, by being resilient, fit the head of the user, and some kind of sound-absorbing material arranged inside the cap shell 13a, 13b. The caps 11 a, 11 b are also commonly provided with a so-called bottom plate 15a (only visible at the user's left ear). The bottom plate 15a is positioned between the sealing ring 14a and the cap shell 13a and connects the sealing ring 14a to the cap shell 13a. The bottom plate 15a also commonly acts to keep the sound-absorbing material in place inside the cap shell 13a.

As shown in fig 2, the first portion 6, is adapted to, when a user wears a hearing cap, be positioned between the sealing ring 14a of the hearing cap 13a and a portion of the user's head circumscribing an ear. The first portion 6 is in contact with the sealing ring 15a of the hearing cap. The first portion is further in contact with the user's head around the user's external ear.

The first portion 6 comprises a relatively air tight material forming a sealing ring in the headpiece. Suitable materials are textiles laminated with a tight layer, such as polymer materials, tight woven textiles, EPDM (ethylene propylene diene monomer) foam (collapsing foam with closed cells), non-woven, laminated non-woven, homogenous polymer films or foils.

The first portion 6 is formed of a material being rigid in the direction of the normal of the material. A thin foil of a rigid polymer material will for example be rigid in the direction of the normal but due to the thin shape still be bendable. The material may alternatively be made comparably rigid by being formed of a thin foil or the like of an otherwise considered elastic material. By making it thin the elasticity is in such a case negligible.

The second portion 7 is formed of one or more relatively air permeable materials allowing air to move between the ear and an inner volume of the hearing cap when the user wears the hearing cap.

The second portion 7 should also be able to be flexible to accommodate the external ear's profile such that the sealing ring formed by the first portion 6 remains flat.

When the headpiece 1 is worn without any hearing cap is should also prevent the ear from being subjected to cold, wind and rain. Still it is advantageous if the material breaths such that it releases sweat and heat.

Suitable materials are combinations of an open material allowing air flow and an flexible, elastic material allowing deformation. Such materials may for example be elastic textiles, elastic nets, elastic non-woven, and perforated plastic films.

The materials forming the headpiece should preferably be soft or formable during normal use, i.e. they should be able to be soft such that they follow the users head in the desired manner. Normal temperatures of use extend from about -35°C to about +40°C. It may be noted that it is especially suitable if the first portion 6 is formable such that it will form a tight sealing with the user's head. It may also be noted that even if the first portion 6 is formed of a material being too rigid such that it at low temperatures does not really follow the user's head, it will in most cases quickly be heated by the user's body heat, thereby reaching a temperature at which it is formable enough.

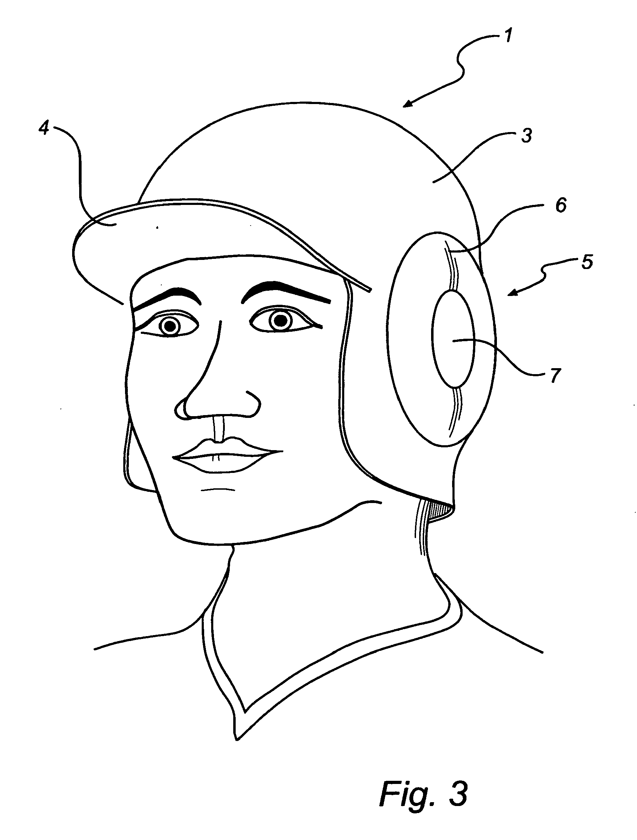

Fig 3 discloses a headpiece 1 in accordance with a second embodiment in the form of a cap provided with ear flaps. In comparison with the headpiece of fig 1 and fig 2 it is basically the neck portion missing. The headpiece 1 is also provided with a first portion 6 and a second portion 7 as discussed in detail in relation to fig 1 and fig 2.

The neck portion 2 and head portion 3, i.e. the headpiece 1 as such has basically two functions; firstly to keep the ear portions 5 in place and secondly to protect the user's head. It may protect the user's head against sun, rain, wind, cold or other natural or manmade environmental conditions. When used to protect against sun it may e.g. be made of a cool and thin but sun-blocking material. When used to protect against cold, rain, wind it may e.g. be made of a warm, thick, and wind tight material.

The materials may be different kinds of textiles, non-woven materials, or films or foils that are capable of being shaped after the user's head.

The headpiece may also be used with other protective pieces such as a helmet, protective glasses or the like. The headpiece may e.g. be provided with a portion fitting snugly with protective glasses. The headpiece may also be provided as an integrated or removable insert in a helmet.

### Detailed analysis of the graphs

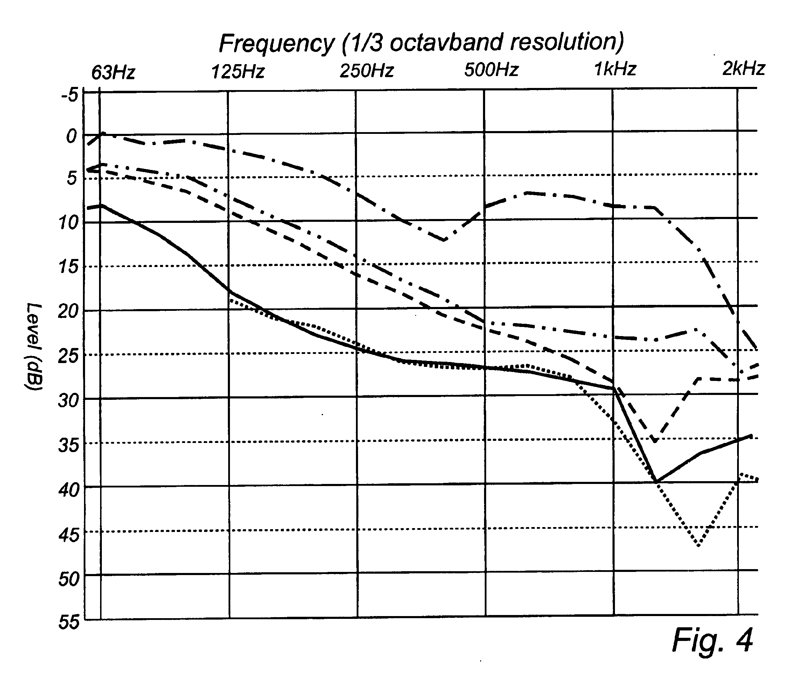

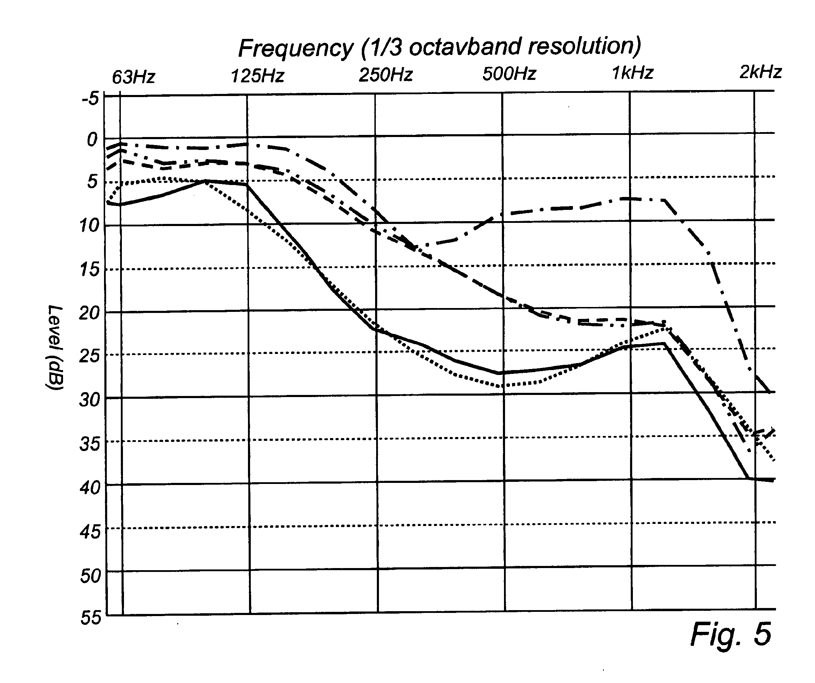

Fig 4 and fig 5 show for two different hearing caps the sound attenuation capacity in the frequency range from 63Hz to 2kHz of respective hearing cap as such and the respective hearing cap worn in combination with different headpieces.

The tests are conducted by putting a hearing cap onto a persons head with one microphone inside the persons ear and one microphone on the outside of the hearing protection. Sound is emitted for the different frequencies studied and the difference between the registration of the two microphones is plotted as the graphs in fig 4 and fig 5.

In fig 4 and fig 5 the sound attenuation is indicated by a negative number (level dB) on the y-axis. The greater sound attenuation, i.e. the greater difference between the registration of sound outside and inside the hearing protection, the greater negative value on the y-axis.

In fig 4 the dotted line indicates a first hearing cap as such when worn with the sealing ring directly abutting the user's head around the outer ear of the user. Similarly, in fig 5 the dotted line indicates a second hearing cap as such when worn with the sealing ring directly abutting the user's head around the outer ear of the user.

In both fig 4 and fig 5 three different dash or dash-dotted lines extend clearly above the dotted line. The upper-most, dash-dotted line represents the sound attenuation when the hearing cap is worn outside a fleece cap. The second upper-most, dash-double-dotted line represents the sound attenuation when the hearing cap is worn outside a knitted cap. The third upper-most, dashed line represents the sound attenuation when the hearing cap is worn outside a ski cap.

In fig 4 and fig 5 there is also a solid line extending basically alongside the dotted line. This solid line represents the sound attenuation when the hearing cap is worn outside a ground crew cap of fig 1, i.e. a headpiece in accordance with the invention.

The lines indicating the sound attenuation when the hearing protection is worn outside different types of ordinary headpieces, the knitted cap, the fleece cap and the ski cap, are all above the line of the hearing protection as such and all shows that the sound attenuation is severely worsened compared to the hearing protection as such. Thus, if a user wears the hearing protection outside, for example, a fleece cap, he/she risk seriously damaging his/her hearing. The sound attenuation is only a fraction of the sound attenuation of the hearing protection when worn as such.

However, as shown by the solid line the sound attenuation capacity is relatively unchanged when the hearing cap is worn in combination with the inventive headpiece.

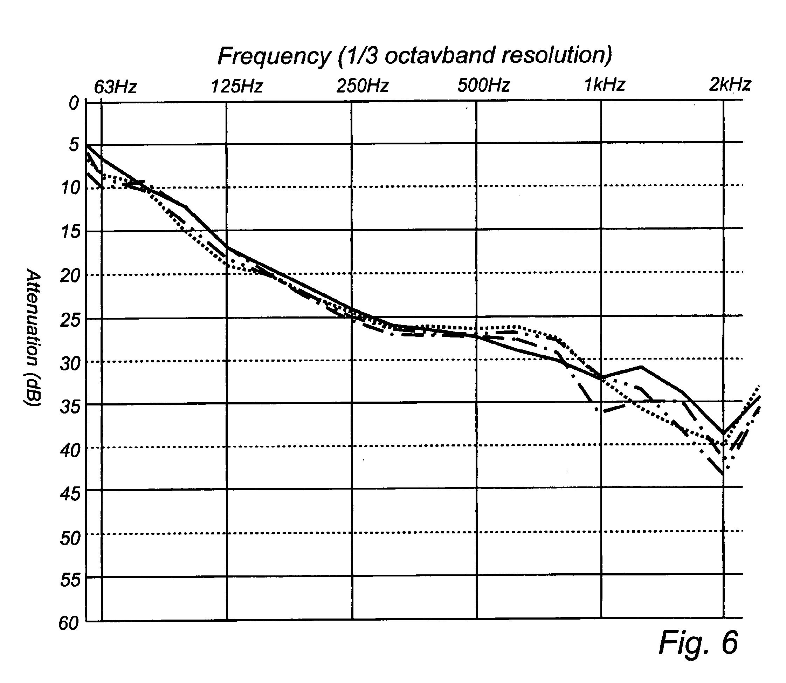

Fig 6 illustrates that when using a PVC laminated textile as the air tight material in the portion of the headpiece abutting the sealing ring of the hearing cap, varying the thickness of the layer in said portion does not result in any major changes in the sound attenuation capacity. In the figure, the sound attenuation capacity with the portion formed of only one layer of PVC laminated textile (0.5 mm) (shown as solid line) is compared to a portion of five layers (2.5 mm) (shown as dash-dotted line), nine layers (4.5 mm) (shown as dash-double-dotted line), and 12 layers (6 mm) (shown as dotted line). These changes in layer thickness, according to figure 6, do not have any significant influence on the sound attenuation capacity.

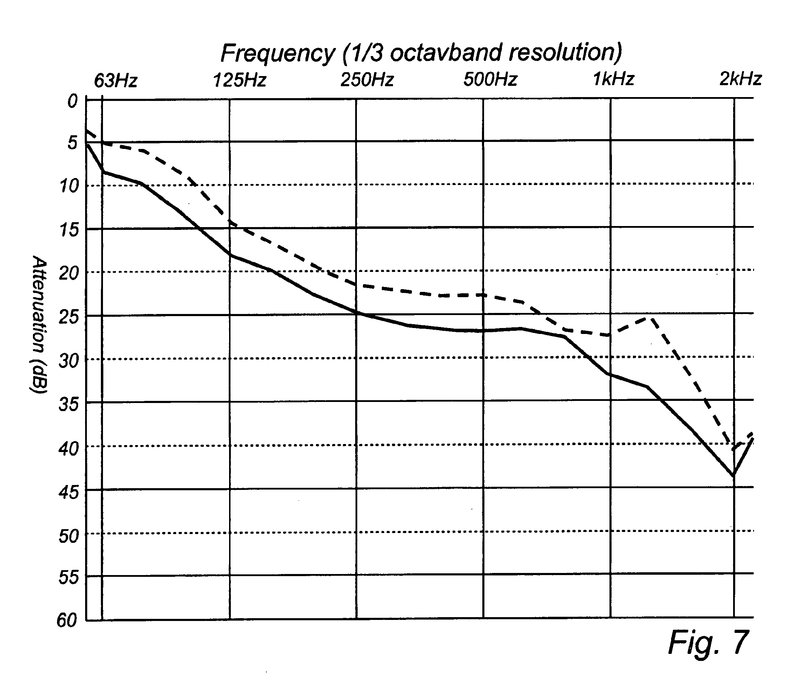

Fig 7 shows the difference between two different air tight materials for the portion abutting the sealing ring of the headpiece, one rigid material (solid line) and one elastic and flexible material (dotted line). The rigid material consist of a 4.5 mm thick layer of PVC laminated textile. The elastic material consist of a 8 mm thick layer of EPDM foam (ethylene propylene diene monomer) which is compressed to a thickness of 4.5 mm but the material is still elastic. Fig 7 shows that the sound attenuation capacity of the hearing cap worn in combination with a headpiece with an elastic material is reduced compared to when the portion consists of a rigid material.

It may be noted that with thin materials, thinner than about 3mm and preferably thinner than 2 mm, the elasticity of the material does not have any significant effect on the sound attenuation. Even an elastic material is comparable non-elastic or rigid if it is thin enough, since the elasticity of material becomes negligible.

If the material positioned between the sealing ring of the hearing cap and a portion of the user's head is elastic, i.e. thick enough and with elastic properties, the material acts as a damper or spring, thereby affecting the sound attenuation and resonance of the hearing cap. The more rigid material, the less influence (even when becoming thicker) it has on the sound attenuation of the hearing cap.

## Claims

1. Headpiece (1) to be worn on a user's head in association with at least one hearing cap (11), **characterised in that** the headpiece (1) comprises
a first portion (6), adapted to when a user wears a hearing cap (11), be positioned between a sealing ring (14) of the hearing cap (11) and a portion of the user's head circumscribing an ear, and
a second portion (7) essentially circumscribed by the first portion (6),
the first portion (6) comprising a relatively air tight material forming a
sealing ring in the headpiece, and
the second portion (7) being formed of one or more relatively air
permeable materials allowing air to move between the ear and an inner volume of the hearing cap (11) when the user wears the hearing cap (11) in association with the headpiece (1).

2. Headpiece (1) according to claim 1, wherein said first portion (6) and said second portion (7) form an ear portion (5), and wherein said headpiece (1) comprises two ear portions (5), one for each ear of the user.

3. Headpiece (1) according to one or more of claims 1-2, wherein the first portion (6), forms a closed loop.

4. Headpiece (1) according to one or more of claims 1-3, wherein the first portion (6) is adapted to, when a user wears the hearing cap (11), be in contact with the sealing ring (14) of the hearing cap (11).

5. Headpiece (1) according to one or more of claims 1-4, wherein the first portion (6) is formed of a rigid material.

6. Headpiece (1) according to one or more of claims 1-4, wherein the first portion (6) is formed of a thin foil of an otherwise elastic material, such that the first portion is rigid.

7. Headpiece (1) according to one or more of claims 1-6, wherein the first portion (6) is air tight such that the acoustic properties of the hearing cap (11) is substantially maintained compared to the hearing cap (11) when worn without the headpiece (1).

8. Headpiece (1) according to one or more of claims 1-7, wherein the second portion (7) being formed of one or more flexible, preferably elastic, materials adapted to, when a user wears the headpiece (1), accommodate the user's ear.

9. Headpiece (1) according one or more of claims 1-8, wherein the second portion (7) is air permeable such that the acoustic properties of the hearing cap (11) is substantially maintained compared to the hearing cap (11) when worn without the headpiece (1).

## Patentansprüche

1. Kopfbedeckung (1), die auf dem Kopf eines Benutzers zu tragen ist, in Verbindung mit wenigstens einem Gehörschutz (11), **dadurch gekennzeichnet, dass** die Kopfbedeckung (1) Folgendes umfasst:
einen ersten Abschnitt (6), der dafür eingerichtet ist, wenn ein Benutzer einen Gehörschutz (11) trägt, zwischen einem Dichtungsring (14) des Gehörschutzes (11) und einem ein Ohr begrenzenden Abschnitt des Kopfes des Benutzers angeordnet zu werden, und
einen zweiten Abschnitt (7), der im Wesentlichen durch den ersten Abschnitt (6) begrenzt wird,
wobei der erste Abschnitt (6) einen verhältnismäßig luftdichten Werkstoff umfasst, der einen Dichtungsring in der Kopfbedeckung bildet, und
wobei der zweite Abschnitt (7) aus einem oder mehreren verhältnismäßig luftdurchlässigen Werkstoffen geformt ist, die es ermöglichen, dass sich Luft zwischen dem Ohr und einem inneren Volumen des Gehörschutzes (11) bewegt, wenn der Benutzer den Gehörschutz (11) in Verbindung mit der Kopfbedeckung (1) trägt.

2. Kopfbedeckung (1) nach Anspruch 1, wobei der erste Abschnitt (6) und der zweite Abschnitt (7) einen Ohrenabschnitt (5) bilden, und wobei die Kopfbedeckung (1) zwei Ohrenabschnitte (5), einen für jedes Ohr des Benutzers, umfasst.

3. Kopfbedeckung (1) nach einem oder mehreren der Ansprüche 1 bis 2, wobei der erste Abschnitt (6) einen geschlossenen Kreis bildet.

4. Kopfbedeckung (1) nach einem oder mehreren der Ansprüche 1 bis 3, wobei der erste Abschnitt (6) dafür eingerichtet ist, wenn ein Benutzer den Gehörschutz (11) trägt, sich in Berührung mit dem Dichtungsring (14) des Gehörschutzes (11) zu befinden.

5. Kopfbedeckung (1) nach einem oder mehreren der Ansprüche 1 bis 4, wobei der erste Abschnitt (6) aus einem steifen Werkstoff geformt ist.

6. Kopfbedeckung (1) nach einem oder mehreren der Ansprüche 1 bis 4, wobei der erste Abschnitt (6) aus einer dünnen Folie eines ansonsten elastischen Werkstoffs geformt ist derart, dass der erste Abschnitt (6) steif ist.

7. Kopfbedeckung (1) nach einem oder mehreren der Ansprüche 1 bis 6, wobei der erste Abschnitt (6) luftdicht ist derart, dass die akustischen Eigenschaften des Gehörschutzes (11), verglichen mit dem Gehörschutz, wenn er ohne die Kopfbedeckung (1) getragen wird, im Wesentlichen beibehalten werden.

8. Kopfbedeckung (1) nach einem oder mehreren der Ansprüche 1 bis 7, wobei der zweite Abschnitt (7) aus einem oder mehreren flexiblen, vorzugsweise elastischen, Werkstoffen geformt ist, die dafür eingerichtet sind, wenn ein Benutzer die Kopfbedeckung (1) trägt, das Ohr des Benutzers aufzunehmen.

9. Kopfbedeckung (1) nach einem oder mehreren der Ansprüche 1 bis 8, wobei der zweite Abschnitt (7) luftdurchlässig ist derart, dass die akustischen Eigenschaften des Gehörschutzes (11), verglichen mit dem Gehörschutz, wenn er ohne die Kopfbedeckung (1) getragen wird, im Wesentlichen beibehalten werden.

## Revendications

1. Casque (1) destiné à être porté sur la tête d'un utilisateur en association avec au moins un capuchon d'audition (11), **caractérisé en ce que** le casque (1) comprend :
une première partie (6) qui s'adapte lorsqu'un utilisateur porte un capuchon d'audition (11), à être positionnée entre une bague d'étanchéité (14) du capuchon d'audition (11) et une partie de la tête de l'utilisateur entourant l'oreille, et
une seconde partie (7) essentiellement délimitée par la première partie (6),
la première partie comprenant un matériau relativement étanche à l'air formant une bague d'étanchéité dans le casque, et
la seconde partie (7) formée d'une ou plusieurs matières relativement perméables à l'air permettant à l'air de circuler entre l'oreille et un volume intérieur du capuchon d'audition (11) lorsque l'utilisateur porte le capuchon d'audition (11) avec le casque (1).

2. Casque selon la revendication 1, où ladite première partie (6) et ladite seconde partie (7) forment une partie de l'oreille (5) et ledit casque (1) comprend deux parties des oreilles (5), une pour chaque oreille de l'utilisateur.

3. Casque (1) selon une ou plusieurs des revendications entre 1-2, où la première partie (6) forme une boucle fermée.

4. Casque (1) selon une ou plusieurs des revendications entre 1-3, où la première partie (6) est adaptée de telle sorte que lorsque l'utilisateur porte le capuchon d'audition (11), elle est en contact avec la bague d'étanchéité (14) du capuchon d'audition (11).

5. Casque (1) selon une ou plusieurs des revendications entre 1-4, où la première partie (6) est formée d'un matériau rigide.

6. Casque (1) selon une ou plusieurs des revendications entre 1 à 5 où la première partie (6) est formée d'une feuille mince d'un matériau non élastique, de telle sorte que la première partie (6) est rigide.

7. Casque (1) selon une ou plusieurs des revendications entre 1 à 6 où la première partie (6) est étanche à l'air de telle sorte que les propriétés acoustiques du capuchon d'audition (11) sont sensiblement maintenues par rapport au capuchon d'audition (11) porté sans casque (1).

8. Casque (1) selon une ou plusieurs des revendications entre 1 à 7 où la seconde partie (7) est formée d'un ou de plusieurs matériaux souple(s), de préférence élastique(s), adapté(s) de telle sorte que lorsqu'un utilisateur porte le casque (1), il(s) s'ajuste(nt) à son oreille.

9. Casque (1) selon une ou plusieurs des revendications entre 1 à 8 où la seconde partie (7) est perméable à l'air de telle sorte que les propriétés acoustiques du capuchon d'audition (11) sont sensiblement maintenues par rapport au capuchon d'audition (11) porté sans casque (1).
